# EUROPÄISCHE PATENTSCHRIFT

(11) **EP 1 265 646 B1**
(45) Veröffentlichungstag und Bekanntmachung des Hinweises auf die Patenterteilung: **19.11.2003**
(21) Anmeldenummer: 01933574.4
(22) Anmeldetag: 22.03.2001
(51) Int. Cl.: A61L 9/12, B05B 7/24

(54) **DÜSE FÜR DIE ABGABE VON WIRKSTOFFEN**
NOZZLE FOR DISSEMINATING ACTIVE SUBSTANCES
BUSE DE DISTRIBUTION D'AGENTS ACTIFS

(30) Priorität: 24.03.2000 DE 10014339; 12.07.2000 DE 10033917
(43) Veröffentlichungstag der Anmeldung: 18.12.2002
(73) Patentinhaber: Aerome GmbH Scent System Engineering, 40221 Düsseldorf (DE)
(72) Erfinder: MEIRE, Marc, 50226 Frechen (DE)
(74) Vertreter: Tönhardt, Marion, Dr.
(86) Internationale Anmeldenummer: DE0101104
(87) Internationale Veröffentlichungsnummer: WO01072346

(56) Entgegenhaltungen:
- DE-A- 19 712 585
- DE-U- 9 402 500
- DE-U- 29 815 783
- FR-A- 2 771 930
- GB-A- 1 286 051
- US-A- 4 707 338
- US-A- 5 050 798
- PATENT ABSTRACTS OF JAPAN vol. 1997, no. 09, 30. September 1997 (1997-09-30) & JP 09 131392 A (BIO MEDEIKU KENKYUSHO:KK;RUMINASU:KK), 20. Mai 1997 (1997-05-20)
- PATENT ABSTRACTS OF JAPAN vol. 016, no. 295 (C-0957), 30. Juni 1992 (1992-06-30) & JP 04 079962 A (KAO CORP), 13. März 1992 (1992-03-13)

## Beschreibung

Die vorliegende Erfindung betrifft eine Düse für die Abgabe von Wirkstoffen, mit einem Düsenkopf, welcher eine Austrittsöffnung für den Wirkstoff hat; und einem Düsenkörper, welcher einen Anschluß für eine Luftleitung aufweist.

Verkaufspräsentationen werden oftmals durch Duftuntermalung unterstützt. Auch Systeme zur Raumbeduftung sind bekannt, selbst Kraftfahrzeuge werden mittlerweile gelegentlich beduftet, um eine für den Fahrer angenehme Atmosphäre zu schaffen.

Die Beduftung kann dabei kontinuierlich geschehen, oftmals ist es aber auch gewünscht, daß die Duftabgabe auf Anforderung erfolgt.

Aus der US 6 004 516 A ist eine Vorrichtung zum Erzeugen eines Duftes auf ein elektronisches Signal hin bekannt. Dabei trägt eine Diskette ein mit Aromen imprägniertes absorbierendes Material. Die Diskette wird in ein entsprechendes Laufwerk eingeführt, das Signale von einem Benutzer oder aber auch von einem Servercomputer erhält. Ein Controller im Laufwerk liefert ein elektrisches, mechanisches oder thermisches Signal an die Diskette, wodurch der Duftstoffträger aufgeheizt wird und der Duft abgegeben wird. Die Duftabgabe kann dabei von einem Gebläse unterstützt werden.

Kritisch bei solchen Duftabgabegeräten sind immer die Duftspeichermedien. Zum einen müssen sie ausreichend Duftstoff oder einen anderen Wirkstoff halten können, damit sich das Speichermedium nicht zu schnell erschöpft. Die thermische Aktivierung des Duft- oder Wirkstoffes birgt weiter das Risiko, diesen zu zerstören oder zumindest zu verfälschen.

Die DE 197 53 956 A1 offenbart eine Wirkstoffkartusche, die von einem Trägergasstrom durchströmt wird, wobei dieses bis zu 1000 Mal geschehen kann, ohne daß die Abgabemenge der Duft- oder Wirkstoffe pro Durchströmung merklich abnimmt. Dazu ist in einem zylinderförmigen Glas- oder Aluminiumröhrchen ein Substrat aus mit den Duft- oder Wirkstoffen imprägnierten porösen Teilchen aus Kieselgel, Aktivkohle und/oder Aluminiurnoxid untergebracht und fixiert. Das Röhrchen ist mit Kappen verschlossen, welche jeweils eine Membranscheibe aufweisen, die für die Freisetzung der Duft- oder Wirkstoffe mit Nadeln oder Kanülen durchstoßen werden können. Diese Duft- oder Wirkstoffekartusche hat sich in der Praxis bewährt.

Die GB-A-1 286 051 offenbart eine Düse mit einem Düsenkopf, der eine Austrittsöffnung für den Wirkstoff hat, einem Düsenkörper, der einen Anschluß für eine Luftleitung hat und aus einer langgestreckten Röhre zur Aufnahme einer Wirkstoffkartusche besteht, wobei in deren ersten Endbereich der Anschluß für die Luftleitung vorgesehen ist. Der Düsenkopf ist zumindest teilweise am zweiten Endbereich der Röhre lösbar in diese einzusetzen und hat einen sich von der Austrittsöffnung in Richtung Wirkstoffkartusche erstreckenden Strömungskanal. Der Düsenkopf dient gleichzeitig als Anlage für die Kartusche.

Es ist die Aufgabe der vorliegenden Erfindung, eine Düse der eingangs genannten Art so zu verbessern, daß in Kartuschen gehaltener Wirkstoff in einfacher Weise durchströmt und mit ausgetragen werden kann. Dabei soll die Art der Wirkstoffkartuschen nicht auf die nach der DE 197 53 956 A1 beschränkt sein.

Es ist auch Aufgabe der Erfindung, ein System mit dieser Düse zum Einspeisen von Wirkstoffen in einen Raum zur Verfügung zu stellen.

Diese Aufgabe wird bei der oben angegebenen Düse dadurch gelöst, daß die Wirkstoffkartusche in Richtung auf den Düsenkopf federbelastet ist. Dies geschieht zweckmäßigerweise dadurch, daß im ersten Endbereich der Röhre eine Spiralfeder gehalten ist, die gegen die Wirkstoffkartusche drückt.

Erfindungsgemäß wird die Wirkstoffkartusche fest in der Düse in einer Lage so gehalten, daß die aus der Luftleitung in die Düse eintretende Luft diese durchströmen kann. In der einfachsten Form würde eine solche Wirkstoffkartusche aus einem an beiden Enden offenen Röhrchen bestehen, wobei das Röhrchen vor Gebrauch versiegelt wäre. Die erfindungsgemäße Anordnung verhindert das Verkanten, die Kartusche, wenn sie erschöpft ist, kann leicht ausgewechselt werden, indem der Düsenkopf abgenommen wird.

Nach einer bevorzugten Ausgestaltung weist die Röhre im ersten Endbereich eine erste Anlageschulter für die Wirkstoffkartusche auf, die eine Bewegungsbegrenzung beim Einführen der Wirkstoffkartusche bildet.

Es kann im ersten Endbereich der Röhre ein Einsatz angeordnet sein, welcher eine als Strömungskanal ausgebildete Hohlnadel hält, wobei ein Ende der Hohlnadel in die Wirkstoffkartusche ragt und das andere Ende mit dem Anschluß kommuniziert. Hierdurch wird die als Trägergas arbeitende Luft direkt an den Wirkstoff herangeführt. Vorteilhaft ist diese Ausgestaltung aber insbesondere, wenn eine anfangs mit einer Membran verschlossene Wirkstoffkartusche verwendet wird. Beim Einsetzen der Wirkstoffkartusche durchsticht die Hohlnadel dann nämlich die Membran und öffnet sie somit für den Luftstrom. Auf bekannte Weise kann dabei die Hohlnadel mit einem abgeschrägten bzw. angespitzten Ende versehen sein.

Im ersten Endbereich der Röhre ist bei dieser Ausgestaltung vorzugsweise eine zweite Anlageschulter vorgesehen, auf der der Einsatz sitzt.

Diese Ausgestaltung kann genutzt werden, die Spiralfeder zwischen Einsatz und Innenwand der Röhre zu halten.

Entsprechend kann der Strömungskanal des Düsenkopfes zumindest teilweise als Hohlnadel ausgebildet sein, welche sich in die Wirkstoffkartusche erstreckt.

Wenn es die Gegebenheiten im zu beduftenden oder mit Wirkstoffen zu beaufschlagenden Raum erfordern, kann der Strömungskanal im Inneren des Düsenkopfes zumindest abschnittsweise mit einem Winkel zur Längsachse des Düsenkopfes verlaufen. Dann ist es zweckmäßig, den Düsenkopf im Hinblick auf einen hinreichenden Duftkegel zu optimieren.

Die Länge des abgewinkelten Strömungskanals zur Austrittsöffnung hin scheint dabei neben der geometrischen Ausführung des Übergangsbereichs entscheidend. Um eine hinreichende Länge des gerade verlaufenden Teils sicherzustellen, wird bevorzugt der Düsenkopf in seinem aus der Röhre ragenden Bereich halbkugelartig ausgebildet.

Das einfache Lösen des Düsenkopfes und das Austauschen der Wirkstoffkartusche gelingt, wenn der Düsenkopf mittels eines Bajonettverschlusses oder zwei Schrauben relativ zu der Röhre gehalten ist.

Das erfindungsgemäße System zum Einspeisen von Wirkstoffen in einen Raum verwendet die Düse zusammen mit einer Pumpe oder einem Kompressor zum Beaufschlagen der in dem Düsenkörper befindlichen Wirkstoffkartusche, wobei die Pumpe oder der Kompressor ansprechend auf das Signal eines Sensors ein- und ausschaltet.

Vorteilhaft gibt der Sensor die Signale an eine elektronische Schaltung, welche den Arbeitsstrom der Pumpe oder des Kompressors steuert.

Besonders bevorzugt ist die elektronische Schaltung fernprogrammierbar, beispielsweise mittels eines Handsenders.

Als Sensor sind Infrarot-, Ultraschall-, Ultrakurzwellen-, Licht- oder Erschütterungssensoren sowie auf Schall ansprechende Sensoren oder eine Kontaktmatte denkbar.

Die elektronische Schaltung kann so ausgelegt sein, daß sie den Wirkstoffstrom bzw. Duftstrom mit einer vorwählbaren Verzögerung auslöst.

Im folgenden soll die Erfindung anhand der beigefügten Zeichnung näher erläutert werden. Es zeigt:
- Figur 1: eine Düse gemäß einer ersten Ausführungsform der vorliegenden Erfindung mit angeschlossenem Luftsystem;
- Figur 2: eine Abwandlung beim Luftanschluß für die Düse;
- Figur 3: eine weitere Ausgestaltung einer Düse gemäß der Erfindung mit abgewandeltem Düsenkopf; und
- Figur 4: in schematischer Darstellung die Steuerung für das Luftsystem.

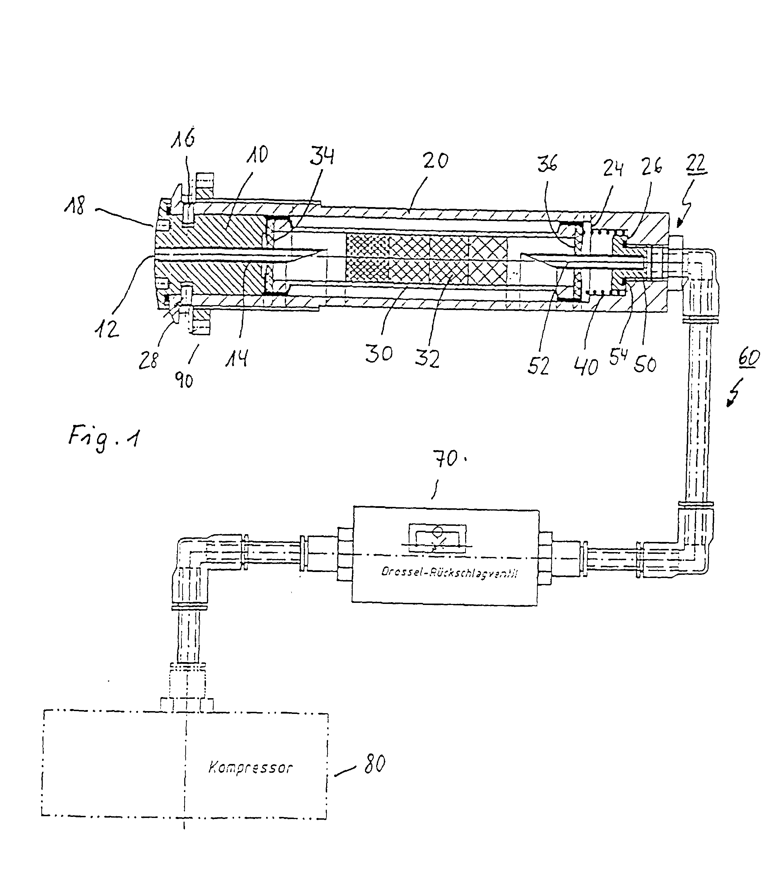

Die in Figur 1 dargestellte Düse weist einen Düsenkopf 10 auf, der teilweise in einer langgestreckten Röhre 20 versenkt ist und an dieser mittels eines nicht näher dargestellten Bajonettverschlusses 16 gehalten wird. Eine Hohlnadel 14 durchsetzt den Düsenkopf 10 und mündet in einer Austrittsöffnung 12. Am gegenüberliegenden Ende erstreckt sich die Hohlnadel 14 über den Düsenkopf 10 hinaus in eine in der Röhre 20 liegende Wirkstoffkartusche 30. Gegen unbefugtes Herausnehmen wird der Düsenkopf 10 durch das Erfordernis eines Spezialschlüssels geschützt. Dieser Spezialschlüssel greift in zwei oder mehr Bohrungen 18, die an der Stirnseite des Düsenkopfes 10 vorgesehen sind.

Der Innendurchmesser der Röhre 20 ist so gewählt, daß eine präzise Führung der Wirkstoffkartusche 30 gewährleistet ist. Die Röhre 20 weist in ihrem dem Düsenkopf 10 gegenüberliegenden Endbereich einen Anschluß 22 für eine Luftleitung 60 auf. Die Ausgestaltung des Anschlusses 22 ist dabei an sich bekannt. Er wird auf übliche Weise in eine Bohrung in der Röhre 20 eingesetzt. Über dem Anschluß 22 sitzt ein Einsatz 50, der von einer Hohlnadel 52 durchsetzt ist. Der Einsatz 50 sitzt mit seinem Kopf auf einer Anlageschulter 26 im Inneren der Röhre 20 und ist mittels eines Dichtringes 54 gegen die Anlageschulter 26 gesetzt, so daß kein Fremdluftaustausch zwischen dem Inneren der Röhre 20 und der Umgebung stattfinden kann. Der auf der Anlageschulter 26 sitzende Teil des Einsatzes 50 ist so bemessen, daß zur Innenwand der Röhre 20 hin ein Raum verbleibt, in dem eine Spiralfeder 40 einliegt. Die Spiralfeder 40 wird somit durch den Einsatz gehalten. Eine entsprechende konische Ausgestaltung der Innenwand, in der Zeichnung nicht dargestellt, unterstützt die Haltefunktion.

Für die Inbetriebnahme der Düse wird bei abgenommenem Düsenkopf 10 eine Wirkstoffkartusche 30 in die Röhre 20 geschoben. Die hier dargestellte Ausführungsform der Düse ist für eine beidseitig mit jeweils einer Membran 34 bzw. 36 verschlossene Wirkstoffkartusche 30 gedacht. Die Röhre wird so weit geschoben, bis die Hohlnadel 52 die Membran 36 durchsticht. Dann wird die Wirkstoffkartusche 30 weiter gegen den Widerstand der Spiralfeder 40 in Richtung auf den Anschluß 22 geschoben, bis sie auf einer Anlageschulter 24 zu liegen kommt, die etwas beabstandet von der Anlageschulter 26 ausgebildet ist. Sodann wird mit Hilfe des schon erwähnten Spezialschlüssels der Düsenkopf 10 in die Röhre 20 gesetzt und mit Hilfe des Bajonettverschlusses 16 verriegelt. Dabei dringt die Hohlnadel 14 durch die Membran 34 in die Wirkstoffkartusche 30. Im allgemeinen genügt nach dem Anstechen der Membran 34 eine Drehung um maximal 180° um den Düsenkopf 10 relativ zur Röhre zu halten, ohne daß eine Zerstörung der Membran 34 zu befürchten wäre. Die Düse ist nun betriebsbereit.

Auf ein Signal hin, das ein Benutzer geben kann, das aber auch von einer anderen Signalquelle erzeugt werden kann, beispielsweise von einer Fernsteuerung, einem mechanischen Taster, einem Bewegungsmelder über das Internet oder dergleichen, wird ein Kompressor 80 beziehungsweise eine Pumpe aktiviert. Der Kompressor 80 ist vorzugsweise auf hohe Lebensdauer bei häufigem Kurzbetrieb auszulegen. Wenn beispielsweise eine herkömmliche Duftpräsentation erfolgen soll, beträgt, beispielsweise in einem Kaufhaus, die tägliche Betriebszeit etwa 12 Stunden, wobei mit 60 Arbeitsintervallen zu maximal 10 Sekunden pro Stunde gerechnet wird. Ein geeigneter Kompressor hat dann eine Lebensdauer von 3 Jahren.

Zwischen Kompressor 80 und die Düse sind eine Drossel und ein Rückschlagventil geschaltet. Bei der dargestellten Ausführungsform sind sie in einem Bauteil 70 integriert, es können aber auch Einzelbauteile vorgesehen sein. Mit der Drossel muß ein Luftstrom zwischen 8 und 12 Litern pro Minute geregelt werden. Das Rückschlagventil verhindert, daß nach Ausschalten des Kompressors 80 ein Luftaustausch zwischen Düse und Kompressor 80 stattfindet.

Wenn nun der Kompressor 80 den erforderlichen Luftstrom erzeugt, gelangt dieser durch die Luftleitung 60 in die Röhre 20, und insbesondere durch die Hohlnadel 52 in die Wirkstoffkartusche 30 und trifft dort auf den Wirkstoff, der auf einem Substrat 32 absorbiert ist. Der Wirkstoff wird in den Luftstrom freigegeben und tritt dann in die Hohlnadel 14 und wird durch die Austrittsöffnung 12 ausgegeben.

Um den Einbau der Düse beispielsweise in Aufzugs- oder Fahrzeugkabinen einzubauen, weist die Röhre 20 einen Ringansatz 28 an seinem Ende auf, in das der Düsenkopf 10 eingesetzt ist. Dieser Ringansatz 28 liegt an der Kabinenwand an, die Düse wird dann rückseitig durch eine Mutter 90 gesichert.

Als alternative Ausgestaltung können auch zwei beabstandete Muttern zum Halten der Düse vorgesehen sein. Der Ringansatz 28 würde dann entfallen.

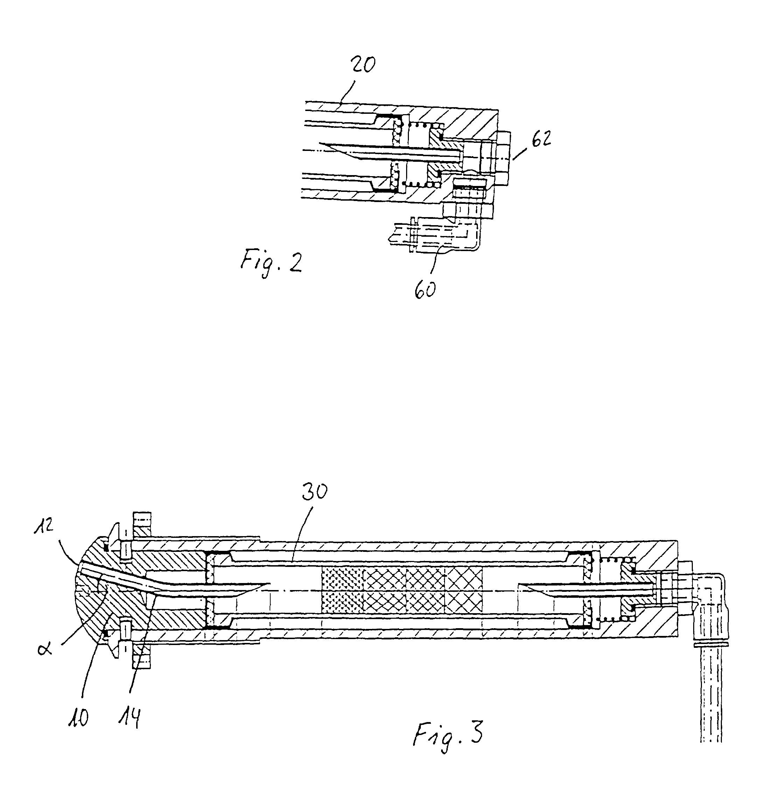

Figur 2 zeigt eine Variante, bei der die Luftleitung 60 radial an der Röhre 20 angeschlossen ist. Ansonsten entspricht der Aufbau dem der Figur 1. Die axiale Bohrung für den Luftanschluß entsprechend der Figur 1 kann mit einer Schraube 62 verschlossen sein.

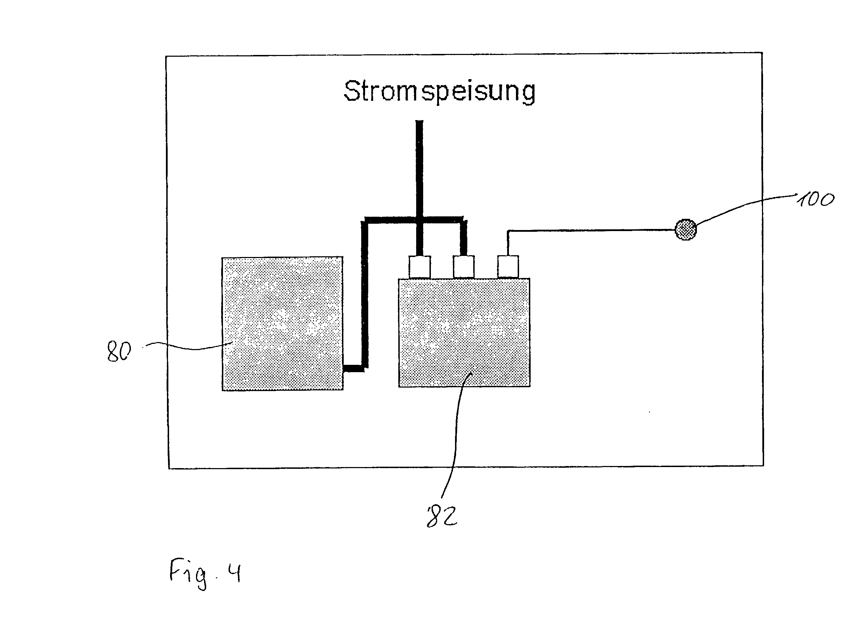

Figur 3 zeigt eine Abwandlung des Düsenkopfes 10. Hierbei ist die Austrittsöffnung 12 nicht mehr auf der Längsachse des Düsenkopfes 10 angeordnet, sondern von dieser versetzt. Der Strömungskanal 14 verläuft dazu unter einem entsprechenden Winkel α zur Längsachse des Düsenkopfes 10, und zwar über eine bestimmte Strecke, die hinsichtlich der Ausbildung eines Duft- oder Wirkstoffkegels zu optimieren ist. Demgemäß würde die Hohlnadel, die den Strömungskanal 14 realisiert, eine Knickstelle aufweisen, damit sie anschließend wieder auf der Längsachse verlaufend in die Wirkstoffkartusche 30 ragen kann. Dazu ist es zweckmäßig, den Düsenkopf 10 nicht massiv zu gestalten, wie es in Figur 1 vorgesehen ist, sondern im Bereich der Knickstelle und des anschließend gerade verlaufenden Teils der Hohlnadel einen Hohlraum vorzusehen, der von einem zylinderartigen Abschnitt 19 der Hülse umschlossen ist. Die Wirkstoffkartusche 30 liegt dann an den zylinderartigen Abschnitt 19 an.

Die Hohlnadel 14 wird zweckmäßigerweise in den Düsenkopf 10 eingeklebt. Der Übergang zwischen Hohlnadel 14 und Austrittsöffnung 12 ist glatt und fugenlos ausgeführt.

Es wird angestrebt, die Gesamtlänge der erfindungsgemäßen Düse so gering wie möglich zu halten. Als optionales Zubehör kann ein Einbauadapter vorgesehen sein, der einen Einbau unter einem bestimmten Winkel ermöglicht, beispielsweise 30°, und zu einem reduzierten Platzbedarf, bei geringerer Einbautiefe führt.

Die Röhre 20 ist bevorzugt aus einem metallischen Werkstoff gefertigt.

Anwendungsbereiche sind nicht nur die bisher angegebenen, bei denen hauptsächlich mit Duftstoffen gearbeitet wird. Es ist beispielsweise auch denkbar, das System mit der erfindungsgemäßen Düse zur Optimierung der Atmosphäre von Räumen zu nutzen, in denen sich beispielsweise Allergiker aufhalten. Dann wird die Kartusche entsprechende Wirkstoffe enthalten.

Figur 4 zeigt in schematischer Darstellung die Steuerung für das Luftsystem. In den Arbeitsstromkreis des Kompressors 80 beziehungsweise der Pumpe ist eine elektronische Schaltung 82 gesetzt, die Signale von einem Sensor 100 empfängt. Der Sensor 100 gibt das Signal bei Bewegung, Annäherung oder Berührung an die elektronische Schaltung 82, welche dann den Kompressor 80 bzw. die Pumpe schaltet und den Wirkstoffstrom auslöst. Dabei sind Auslöseabstand und Dauer des Wirkstoffstroms vorwählbar. Als Sensoren 100 sind Infrarot-, Ultraschall-, Ultrakurzwellen- und Lichtsensoren einsetzbar. Auch auf Schall oder die menschliche Stimme ansprechende Sensoren und Erschütterungssensoren können zum Einsatz kommen. Ebenfalls kann eine Kontaktmatte, die auf dem Boden des Raumes ausgelegt ist, in den die Wirkstoffe eingetragen werden sollen, als Sensor 100 verwendet werden. Die elektronische Schaltung 82 wird mittels eines nicht dargestellten Handsensors in allen Funktionen programmiert. Die elektronische Schaltung 82 kann dabei so ausgelegt sein, daß sie zugleich Benutzungsinformation sammelt, die für Marktforschungszwecke mittels eines Laptops oder Personal Computers abrufbar sind.

Die in der vorstehenden Beschreibung, in der Zeichnung sowie in den Ansprüchen offenbarten Merkmale der Erfindung können sowohl einzeln als auch in beliebiger Kombination für die Verwirklichung der Erfindung wesentlich sein.

## Patentansprüche

1. Düse für die Abgabe von Wirkstoffen, mit
- einem Düsenkopf (10), welcher eine Austrittsöffnung (12) für den Wirkstoff hat; und
- einem Düsenkörper (20), welcher einen Anschluß (22) für eine Luftleitung (60) aufweist,
wobei der Düsenkörper eine langgestreckte Röhre (20) zur Aufnahme einer Wirkstoffkartusche (30) ist, an deren einem Endbereich der Anschluß (22) für die Luftleitung (60) vorgesehen ist, daß der Düsenkopf (10) zumindest teilweise am zweiten Endbereich der Röhre (20) lösbar in diese einzusetzen ist, wobei der Düsenkopf (10) eine Abmessung in axialer Richtung der Röhre (20) hat, daß die Wirkstoffkartusche (30) in Anlage an den Düsenkopf (10) gehalten wird, und der Düsenkopf (10) einen sich von der Austrittsöffnung (12) zur Wirkstoffkartusche (30) erstreckenden Strömungskanal (14) aufweist, **dadurch gekennzeichnet, daß** die Wirkstoffkartusche (30) in Richtung auf den Düsenkopf (10) federbelastet ist.

2. Düse nach Anspruch 1, **dadurch gekennzeichnet, daß** die Röhre (20) im ersten Endbereich eine erste Anlageschulter (24) für die Wirkstoffkartusche (30) aufweist.

3. Düse nach Anspruch 2, **dadurch gekennzeichnet, daß** im ersten Endbereich der Röhre (20) eine Spiralfeder (40) gehalten ist.

4. Düse nach Anspruch 1, **dadurch gekennzeichnet, daß** im ersten Endbereich der Röhre (20) ein Einsatz (50) angeordnet ist, welcher eine als Strömungskanal ausgebildete Hohlnadel (52) hält, wobei ein Ende der Hohlnadel (52) in die Wirkstoffkartusche (30) ragt und das andere Ende mit dem Anschluß (22) kommuniziert.

5. Düse nach Anspruch 4, **dadurch gekennzeichnet, daß** im ersten Endbereich der Röhre (20) eine zweite Anlageschulter (26) vorgesehen ist, auf der der Einsatz (50) sitzt.

6. Düse nach einem der Ansprüche 3 bis 5, **dadurch gekennzeichnet, daß** die Spiralfeder (40) zwischen Einsatz (50) und Innenwand der Röhre (20) gehalten ist.

7. Düse nach Anspruch 1, **dadurch gekennzeichnet, daß** der Strömungskanal (12) des Düsenkopfes (10) zumindest teilweise als Hohlnadel ausgebildet ist, welche sich in die Wirkstoffkartusche (30) erstreckt.

8. Düse nach Anspruch 7, **dadurch gekennzeichnet, daß** der Strömungskanal (12) im Inneren des Düsenkopfes (10) zumindest abschnittsweise mit einem Winkel (α) zur Längsachse des Düsenkopfes (10) verläuft.

9. System zum Einspeisen von Wirkstoffen in einen Raum mit einer Düse nach einem der Ansprüche 1 bis 8, mit einer Pumpe oder einem Kompressor zum Beaufschlagen der in dem Düsenkörper befindlichen Wirkstoffkartusche, **dadurch gekennzeichnet, daß** die Pumpe oder der Kompressor (80) ansprechend auf das Signal eines Sensors (100) ein- und ausschaltet.

10. System nach Anspruch 9, **dadurch gekennzeichnet, daß** der Sensor die Signale an eine elektronische Schaltung (82) gibt, welche den Arbeitsstrom der Pumpe oder des Kompressors (80) steuert.

11. System nach Anspruch 10, **dadurch gekennzeichnet, daß** die elektronische Schaltung (82) fernprogrammierbar ist.

12. System nach einem der Ansprüche 9 bis 11, **dadurch gekennzeichnet, daß** der Sensor (100) ein Infrarot-, Ultraschall-, Ultrakurzwellen-, Licht, Schall- oder Erschütterungssensor ist.

13. System nach einem der Ansprüche 10 bis 12, **dadurch gekennzeichnet, daß** die elektronische Schaltung (82) den Wirkstoffstrom mit einer vorwählbaren Verzögerung auslöst.

## Claims

1. A nozzle for delivering active substances, comprising:
- a nozzle head (10) having an exit aperture (12) for the active substance, and
- a nozzle body (20) having a connection (22) for an air line (60),
the nozzle body being an elongate tube (20) to receive an active substance cartridge (30), at one end zone of which the connection (22) for the air line (60) is provided, in that the nozzle head (10) is adapted to be releasably inserted into the tube (20) at least partially at the second end zone of said tube, the nozzle head (10) having a dimension in the axial direction of the tube (20), in that the active substance cartridge (30) is held in abutment against the nozzle head (10), and the nozzle head (10) has a flow duct (14) extending from the exit aperture (12) to the active substance cartridge (30), **characterised in that** the active substance cartridge (30) is spring-loaded in the direction of the nozzle head (10).

2. A nozzle according to claim 1, **characterised in that** the tube (20) has in the first end zone a first abutment shoulder (24) for the active substance cartridge (30).

3. A nozzle according to claim 2, **characterised in that** a spiral spring (40) is held in the first end zone of the tube (20).

4. A nozzle according to claim 1, **characterised in that** an insert (50) is disposed in the first end zone of the tube (20), said insert (50) holding a hollow needle (52) formed as a flow duct, one end of the hollow needle (52) projecting into the active substance cartridge (30) while the other end communicates with the connection (22).

5. A nozzle according to claim 4, **characterised in that** a second abutment shoulder (26) on which the insert (50) sits is provided in the first end zone of the tube (20).

6. A nozzle according to any one of claims 3 to 5, **characterised in that** the spiral spring (40) is held between the insert (50) and the inner wall of the tube (20) .

7. A nozzle according to claim 1, **characterised in that** the flow duct (12) of the nozzle head (10) is at least partially constructed as a hollow needle extending into the active substance cartridge (30).

8. A nozzle according to claim 7, **characterised in that** the flow duct (12) extends in the interior of the nozzle head (10) at least in portions at an angle (α) to the longitudinal axis of the nozzle head (10).

9. A system for introducing active substances into a room with a nozzle according to any one of claims 1 to 8, with a pump or a compressor for actuating the active substance cartridge in the nozzle body, **characterised in that** the pump or the compressor (60) switches on and off in response to the signal of a sensor (100).

10. A system according to claim 9, **characterised in that** the sensor delivers the signals to an electronic circuit (82) which controls the working current of the pump or of the compressor (80).

11. A system according to claim 10, **characterised in that** the electronic circuit (82) is remote-programmable.

12. A system according to any one of claims 9 to 11, **characterised in that** the sensor (100) is an infrared, ultrasonic, ultrashort wave, light, sound or vibration sensor.

13. A system according to any one of claims 10 to 12, **characterised in that** the electronic circuit (82) releases the active substance flow with a preselectable delay.

## Revendications

1. Buse pour la distribution d'agents actifs, avec
- une tête de buse (10), qui présente une ouverture de sortie (12) pour l'agent actif ; et
- un corps de buse (20) qui présente un raccordement (22) pour une conduite d'air (60),
dans laquelle le corps de buse est un tube (20) étiré en longueur recevant une cartouche d'agent actif (30), sur une zone d'extrémité duquel est prévu le raccordement (22) pour la conduite d'air (60), de telle sorte que la tête de buse (10) peut être insérée au moins partiellement sur la seconde zone d'extrémité du tube (20) de façon amovible dans celle-ci, dans laquelle la tête de buse (10) a une dimension dans la direction axiale du tube (20) telle que la cartouche d'agent actif (30) est maintenue en appui sur la tête de buse (10), et la tête de buse (10) présente un canal d'écoulement (14) s'étendant de l'orifice de sortie (12) à la cartouche d'agent actif (30), **caractérisée en ce que** la cartouche d'agent actif (30) est sollicitée par ressort dans le sens de la tête de buse (10).

2. Buse selon la revendication 1, **caractérisée en ce que** le tube (20) présente dans la première zone d'extrémité un premier épaulement d'appui (24) pour la cartouche d'agent actif (30).

3. Buse selon la revendication 2, **caractérisée en ce qu'**un ressort en spirale (40) est maintenu dans la première zone d'extrémité du tube (20).

4. Buse selon la revendication 1, **caractérisée en ce que** dans la première zone d'extrémité du tube (20) est disposé un insert (50) qui maintient une aiguille creuse (52) formant un canal d'écoulement, une extrémité de l'aiguille creuse (52) dépassant dans la cartouche d'agent actif (30) et l'autre extrémité communiquant avec le raccordement (22).

5. Buse selon la revendication 4, **caractérisée en ce que**, dans la première zone d'extrémité du tube (20), est prévu un second épaulement d'appui (26) sur lequel est placé l'insert (50).

6. Buse selon l'une quelconque des revendications 3 à 5, **caractérisée en ce que** le ressort en spirale (40) est maintenu entre l'insert (50) et la paroi intérieure du tube (20).

7. Buse selon la revendication 1, **caractérisée en ce que** le canal d'écoulement (12) de la tête de buse (10) a au moins partiellement la forme d'une aiguille creuse qui s'étend jusque dans la cartouche d'agent actif (30).

8. Buse selon la revendication (7), **caractérisée en ce que** le canal d'écoulement (12) est décentré à l'intérieur de la tête de buse (10) sur au moins un tronçon, en formant un angle (α) par rapport à l'axe longitudinal de la tête de buse (10).

9. Système pour l'injection d'agents actifs dans un espace avec une buse selon l'une des revendications 1 à 8, avec une pompe ou un compresseur pour l'alimentation de la cartouche d'agent actif se trouvant dans le corps de buse, **caractérisé en ce que** la pompe ou le compresseur (80) se connecte ou se déconnecte en fonction du signal d'un capteur (100).

10. Système selon la revendication 9, **caractérisé en ce que** le capteur envoie les signaux à un circuit électronique (82) qui commande le courant de travail de la pompe ou du compresseur (80).

11. Système selon la revendication 10, **caractérisé en ce que** le circuit électronique (82) est programmable à distance.

12. Système selon l'une des revendications 9 à 11,
**caractérisé en ce que** le capteur (100) est un capteur d'infrarouge, d'ultrasons, d'ondes ultracourtes, de lumière, de son ou de vibrations.

13. Système selon l'une des revendications 10 à 12, **caractérisé en ce que** le circuit électronique (82) déclenche le flux d'agent actif avec une temporisation présélectionnable.
